# EUROPEAN PATENT SPECIFICATION

(11) **EP 2 976 954 B1**
(45) Date of publication and mention of the grant of the patent: **24.08.2016**
(21) Application number: 14815561.7
(22) Date of filing: 30.05.2014
(51) Int. Cl.: A24F 47/00, A61M 15/00, A61M 15/06

(54) **ELECTRONIC ATOMIZATION APPARATUS WITH AIR INLET CAPABLE OF BEING OPENED AND CLOSED**
ELEKTRONISCHE ATOMISIERUNGSVORRICHTUNG MIT EINEM REGULIERBAREN LUFTEINLASS
APPAREIL D'ATOMISATION ÉLECTRONIQUE COMPORTANT UNE ENTRÉE EN MESURE D'ÊTRE OUVERTE ET FERMÉE

(43) Date of publication of application: 27.01.2016
(73) Proprietor: Shenzhen Jieshibo Technology Co. Ltd., Shenzhen, Guangdong 518000 (CN)
(72) Inventor: WU, Jianyong, Shenzhen Guangdong 518000 (CN)
(74) Representative: Sun, Yiming
(86) International application number: PCT/CN2014/078984
(87) International publication number: WO 2015/180160

(56) References cited:
- WO-A1-2013/147492
- WO-A1-2013/151295
- CN-A- 103 462 224
- CN-U- 202 774 132
- CN-U- 203 105 624
- CN-U- 203 407 519
- CN-U- 203 505 590
- KR-A- 20070 096 660
- KR-U- 20110 006 928
- US-A1- 2013 081 642

## Description

### FIELD OF THE INVENTION

The present invention relates to electronic atomization device and, in particular, it concerns an electronic atomization device with an adjustable air inlet.

### BACKGROUND OF THE INVENTION

Electronic atomization device, also called electronic cigarette, atomization tobacco with low temperature, is mainly used for reducing harms brought by smoking traditional cigarette. Its appearance and taste are similar to cigarette and it has more flavors than even normal cigarette does. It can also create a cloud, a taste and a feel that resemble cigarette. The electronic atomization device, which is composed of battery assembly and atomizer, is an imitative cigarette, and flavor components therein can be atomized into gas by atomization so that user can inhale it directly. The flavor components include tobacco liquid, tobacco shred, tobacco powder, tobacco paste, fragrant slice, fragrant bar, liquid tobacco and so on.

The atomization device for atomizing the flavor components generally includes an air inlet for inhaling the outside air which will be mixed with the atomizing gas, and such an air inlet may be an access for the condensate and liquid flavor flowing to the outside world. Generally, the air inlet is arranged at the bottom of the electronic atomization device and cannot be closed when not in use, thus the condensate and liquid flavor are very easy to flow to the outside surface of the electronic atomization device which will cause the outside surface of the electronic atomization device to be dirty. And if the condensate and liquid flavor enter into the inside of the battery assembly via the air inlet, the battery assembly may be damaged.

Various other portable atomizing apparatus are known, namely WO 2013/147492 A1, WO 2013/151295 A1, KR 2011/0006928 U, KR 2007/0096660 A, wherein a rotable ring is disclosed for controlling the air inlet opening.

### SUMMARY OF THE INVENTION

An object of the present invention is to overcome the defects of the prior art by providing an electronic atomization device with an adjustable air inlet, which can prevent the condensate and liquid flavor from flowing out of the electronic atomization device via the air inlet, so as to avoid the outside surface of the electronic atomization device getting dirty and reducing the possibility of damaging the battery assembly.

To achieve the above object, the following technical solutions are provided:
There is provided an electronic atomization device with an adjustable air inlet, which includes a cigarette holder, an atomizer connected to the cigarette holder and a battery assembly for supplying power to the atomizer. The device further includes an air inlet disposed at an upper portion of the atomizer for connecting the atomizer to outside world and a switch assembly disposed between the atomizer and the cigarette holder for opening or closing the air inlet.

Preferably, the switch assembly includes a round ring sandwiched between the atomizer and the cigarette holder and a sealing block fixed inside the round ring for opening or closing the air inlet, the round ring being coaxial with the atomizer, and when the round ring rotates with respect to the atomizer, the air inlet will be opened or closed by the sealing block which is driven by the round ring.

Preferably, there is provided a flange disposed inside the round ring and the sealing block is fixed on the flange.

Preferably, there are provided a limiting notch formed at the flange and a limiting pillar disposed at the top of the atomizer and extending throughout the limiting notch. Due to the assembly relation between the limiting notch and the limiting pillar, the rotation range of round ring will be restricted, thereby avoiding the sealing block far away from the air inlet which will bring inconvenience to use.

Preferably, the round ring has an inlet notch formed at the bottom thereof and the air inlet connects to the outside world via the inlet notch.

Preferably, the round ring has ant-slip strips formed on an outside surface thereof.

Preferably, the sealing block is an elastic sealing block.

Preferably, the sealing block contacts with an upper surface of the air inlet, and the upper surface of the air inlet is an inclined surface so that the sealing block is capable of applying pressing force onto the upper surface of the air inlet when the air inlet is closed completely by means of the sealing block.

Preferably, the switch assembly further includes an elastic pad disposed between the sealing block and the air inlet, and the elastic pad has a through hole corresponding to the air inlet.

Preferably, a concave is formed around the air inlet for positioning the elastic pad and a convex corresponding to the concave is provided at the bottom of the elastic pad, and the through hole extends through the convex. There is friction between the sealing block and the elastic pad during the air inlet being opened or closed, thus the elastic pad needs to be fixed so as to ensure the function of opening and closing the air inlet.

Preferably, the sealing block and the round ring form an integrated body and the sealing block has a blind hole formed on an upper surface thereof. The blind hole is used for distinguishing the installation direction of the round ring when assembling.

Preferably, the atomizer has a limiting cylinder formed on the top thereof and the limiting cylinder has a notch formed thereon for restricting the rotation range of the sealing block.

Preferably, the atomizer has an air passage formed therein, by which the atomizer connects to the air inlet.

Preferably, the air passage is a circular chamber.

Preferably, the cigarette holder includes a smoke channel, which is provided within the cigarette holder and connected to the atomizer, and two smoke outlets connected to the smoke channel, and the smoke outlets are disposed at a sidewall of the cigarette holder.

Preferably, the smoke outlets are disposed at an upper portion of the cigarette holder uniformly. Such a structure will cause that the smokes fill the mouth of user more quickly and easily when using the electronic cigarette. Additionally, it will increase the distance the smokes have to travel in the cigarette holder, thereby lowering the temperature of smokes in the cigarette holder and then improving the taste of the electronic cigarette.

Preferably, an upper portion of the cigarette holder has an elliptical or round cross section. Such an elliptical cross section of the cigarette holder can better meet the requirement of human body engineering, thereby improving the user experience.

Preferably, the battery assembly includes a battery and a voltage adjusting unit which includes an adjustable resistor in series with the battery and an adjusting knob by which a resistance value of the adjustable resistor is capable of being changed so as to adjust the voltage applied on the atomizer.

Preferably, the voltage adjusting unit further includes a connecting sleeve for determining a relative location between the adjustable resistor and the adjusting knob; the adjusting knob has a central shaft for connecting with the adjustable resistor; the central shaft is clamped by the connecting sleeve and capable of rotating with respect to the connecting sleeve and the adjustable resistor is fixed on a circuit board which is clamped by the connecting sleeve.

Preferably, the battery assembly further includes a casing for containing the battery and the voltage adjusting unit further includes a fixed round ring by which the connecting sleeve is fixed to the casing; the fixed round ring is disposed on an outside surface of the connecting sleeve and fixed to an inner surface of the casing by an interference fit.

Compared with the prior art, the present invention has beneficial effects as follows:
As disclosed in the present invention, the electronic atomization device has an air inlet disposed at the upper portion of the atomizer but not at the lower portion thereof, which can reduce the possibility of the condensate and liquid flavor flowing out of the electronic atomization device when the electronic atomization device dumping. Moreover, the air inlet can be closed completely by means of the switch assembly thereby preventing the condensate and liquid flavor from flowing to the outside world, avoiding the outsider surface of the electronic atomization device getting dirty and reducing the possibility of the damage of the battery assembly.

### BRIEF DESCRIPTION OF THE DRAWINGS

FIG. 1 is a perspective view of an electronic atomization device according to first embodiment of the present invention;
FIG. 2 shows an upper portion of an atomizer of the electronic atomization device according to first embodiment of the present invention;
FIG. 3 shows that an air inlet is covered with a sealing block according to first embodiment of the present invention;
FIG. 4 shows that a switch assembly is mounted on the upper portion of the atomizer according to first embodiment of the present invention;
FIG. 5 is a perspective view (from top) of a round ring according to first embodiment of the present invention;
FIG. 6 is a perspective view of the switch assembly according to first embodiment of the present invention;
FIG. 7 is a perspective view (from bottom) of a round ring according to first embodiment of the present invention;
FIG. 8 is a section view of the electronic atomization device according to first embodiment of the present invention;
FIG. 9 is a perspective view of an electronic atomization device according to second embodiment of the present invention;
FIG. 10 is a section view of the electronic atomization device according to second embodiment of the present invention;
FIG. 11 is a section view of a cigarette holder according to second embodiment of the present invention;
FIG. 12 is a perspective view of the cigarette holder according to second embodiment of the present invention;
FIG. 13 is an exploded view of a switch assembly according to second embodiment of the present invention;
FIG. 14 shows the installation process of an elastic pad of the switch assembly according to second embodiment of the present invention;
FIG. 15 is a perspective view (from bottom) of a round ring according to second embodiment of the present invention;
FIG. 16 is a perspective view showing that the air inlet is completely open according to second embodiment of the present invention;
FIG. 17 is a perspective view showing that the air inlet is completely closed by a sealing block according to second embodiment of the present invention;
FIG. 18 is a section view showing a voltage adjusting unit according to second embodiment of the present invention;
FIG. 19 is an exploded view of the voltage adjusting unit according to second embodiment of the present invention; and
FIG. 20 is a perspective view of an adjustable resistor according to second embodiment of the present invention.

### DETAILED DESCRIPTION OF ILLUSTRATED EMBODIMENTS

The present invention is defined in claim 1.

For better understanding the technical content of the present invention, the specific embodiments are provided to introduce and illustrate the technical solution of the present invention as follows.

### The First Embodiment

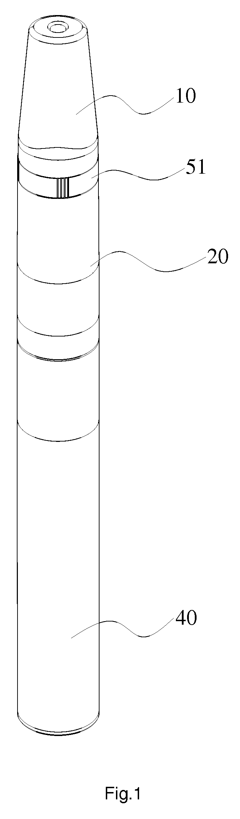

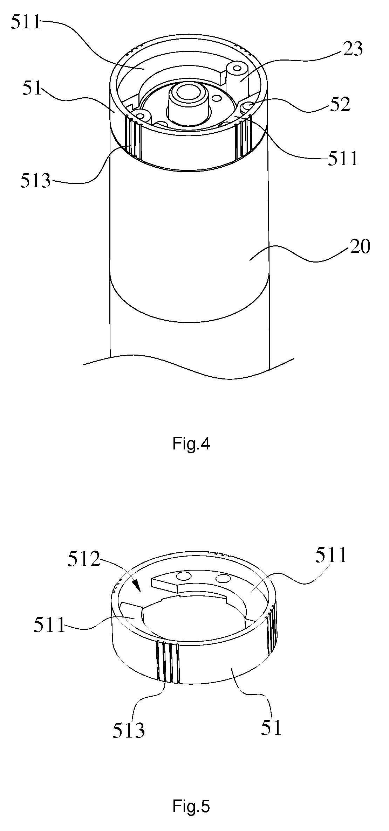

FIG. 1 to FIG. 8 shows the detailed structures of the electronic atomization device according to the first embodiment of the present invention.

The electronic atomization device with an adjustable air inlet of the first embodiment includes a cigarette holder 10, an atomizer 20 connected to the cigarette holder 10 and a battery assembly 40 for supplying power to the atomizer 20.

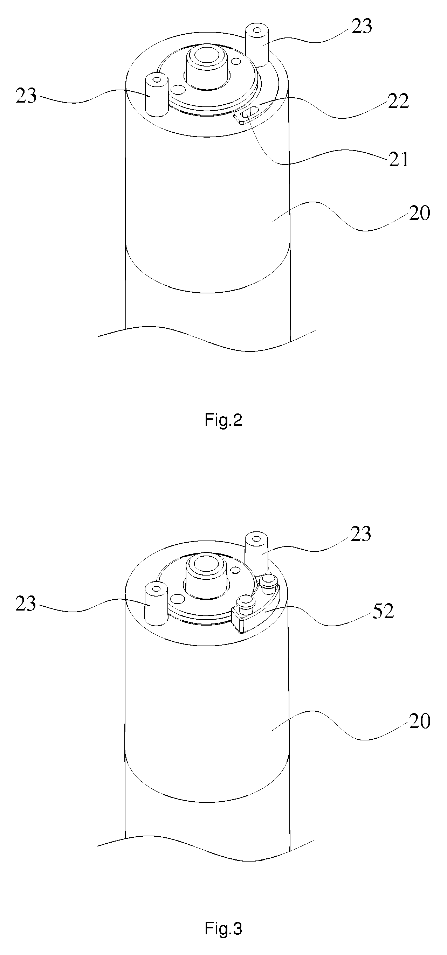

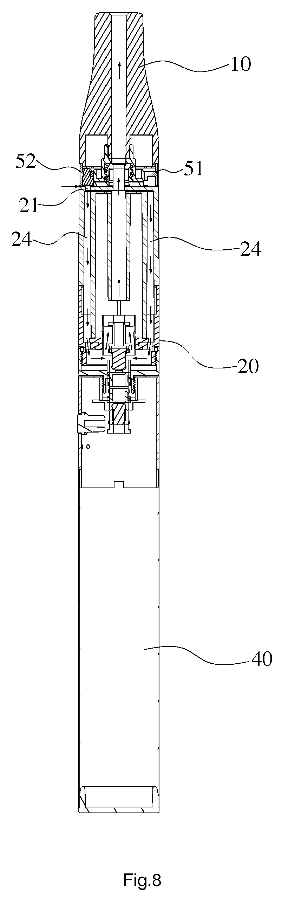

There are provided an air inlet 21 (shown in FIG. 2 and FIG. 8) with an upward opening and disposed at the upper portion of the atomizer 20 and a switch assembly 50 disposed between the atomizer 20 and the cigarette holder 10 for opening or closing the air inlet 21.

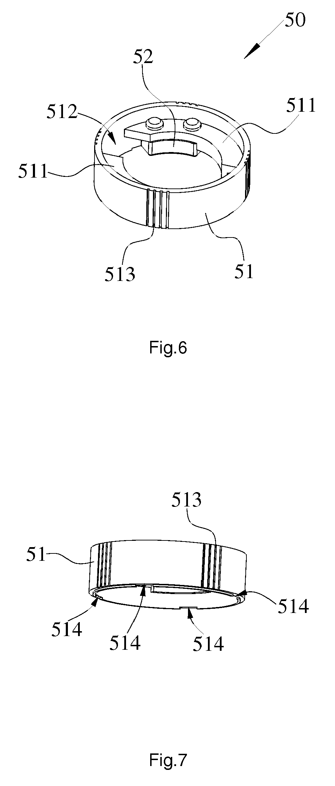

The switch assembly 50 includes a round ring 51 sandwiched between the atomizer 20 and the cigarette holder 10 and a sealing block 52 fixed in the round ring 51 for opening or closing the air inlet 21. The sealing block 52 closely contacts with the upper surface 22 of the air inlet 21. The round ring 51 is coaxial with the atomizer 20. Specifically, the bottom of the round ring 51 contacts with the top of the atomizer 20. The cigarette holder 10 has a stepped shaft disposed at the bottom thereof and the round ring 51 is mounted on the stepped shaft. The round ring 51 has a flange 511 extending from an inner surface thereof radially and the sealing block 52 is fixed on the flange 511 (as shown in FIG. 6).

The atomizer 20 connects to the outside world via the air inlet 21. Specifically, the atomizer 20 has an air passage 24 formed therein, by which the atomizer 20 connects to the air inlet 21 (as shown in FIG. 8), and the round ring 51 has four inlet notches 514 formed at the bottom thereof uniformly, by which the air inlet 21 connects to the outside world (as shown in FIG. 7). In this embodiment, the air passage 24 is a circular chamber.

When using, the user can make the round ring 51 rotate with respect to the atomizer 20, at this time, the exposed area of the air inlet 21 is changed by the sealing block 52 which is driven by the round ring 51, thereby closing or opening the air inlet 21. There are provided anti-slip strips on the outside surface of the round ring 51 so as to avoid slipping when the user turning the round ring 51.

When using, the round ring 51 does not need to make a wide-angle and large-scale rotation, thus it needs to restrict the rotation angle of the round ring 51. There are provided two limiting notches 512 formed at the flange 511 and two limiting pillars 23 disposed at the top of the atomizer 20. And each limiting pillar 23 extends throughout one of the limiting notches 512.

The sealing block 52 is an elastic sealing block which may be made of silica gel. The upper surface 22 of the air inlet 21 is an inclined surface so that the sealing block 52 can produce pressing force onto the upper surface 22 of the air inlet 21 when the air inlet 21 is closed completely by means of the sealing block 52. The lower surface of the sealing block 52 is a surface which contacts with the upper surface 22 of the air inlet 21. The lower surface of the sealing block 52 can be designed to be a flat surface.

### The Second Embodiment

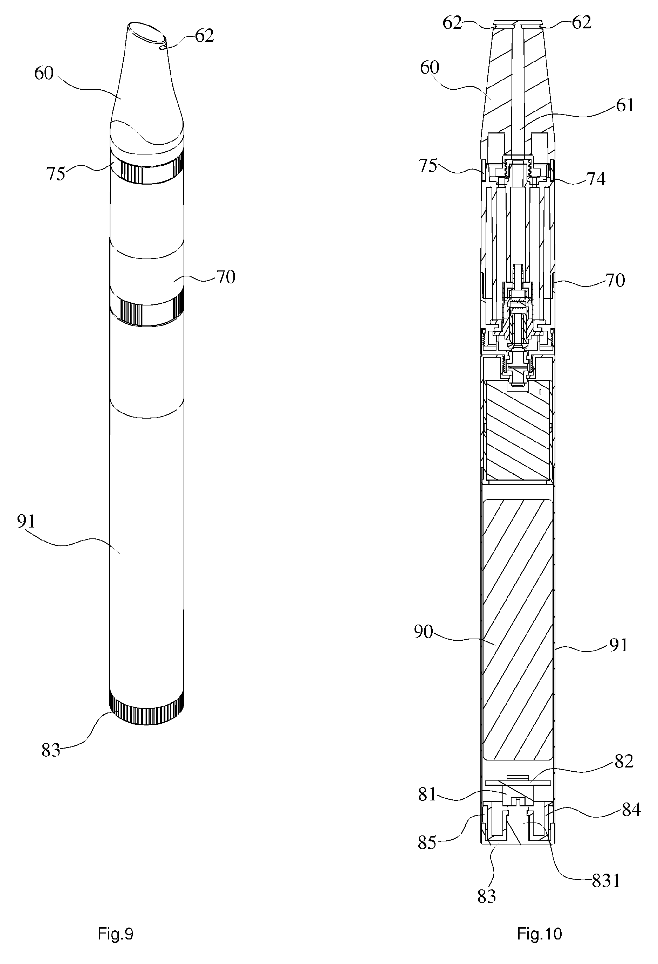

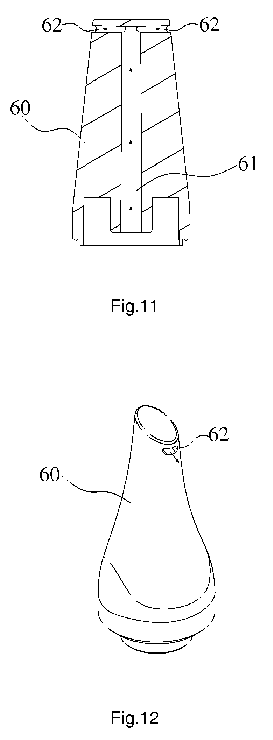

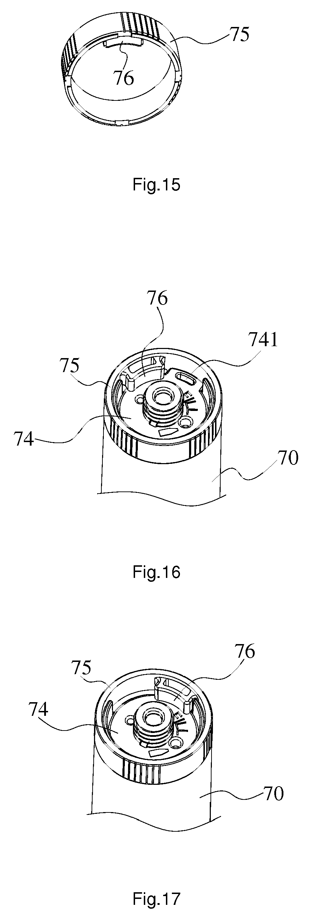

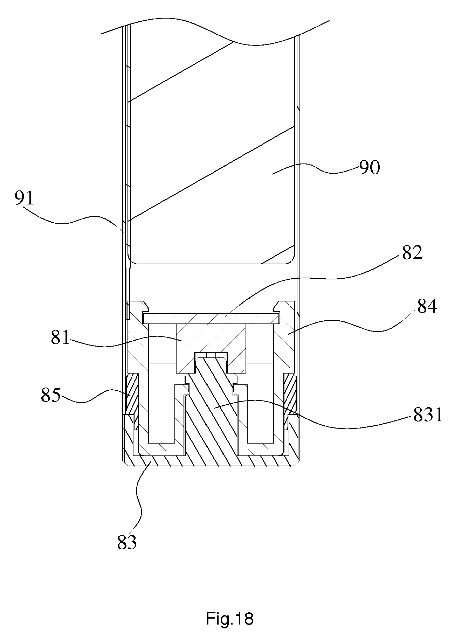

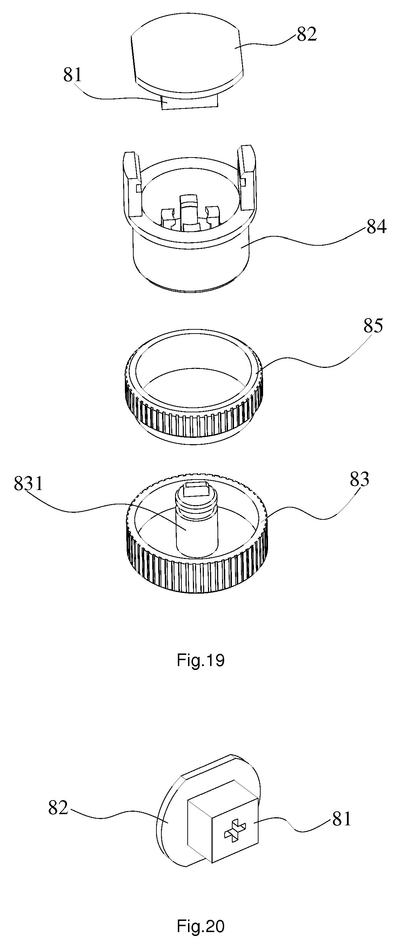

The differences between the second embodiment and the first embodiment are the structures of the cigarette holder, switch assembly and battery assembly, all of which are shown in FIG.9 to FIG. 20.

In the second embodiment, as shown in FIG.9 to FIG. 12, the cigarette holder 60 includes a smoke channel 61, which is provided within the cigarette holder 60 and connected to the atomizer, and two smoke outlets 62 connected to the smoke channel 61 and disposed at a sidewall of the cigarette holder 60. The smoke outlets are disposed at an upper portion of the cigarette holder 60 uniformly. Specifically, the two smoke outlets 62 are disposed at the upper portion of the cigarette holder 60 symmetrically and the upper portion of the cigarette holder 60 has an elliptical cross section. The two smoke outlets 62 are disposed at two intersections between the long axis of the elliptical section and the outside wall of the cigarette holder 60, respectively. In other embodiments, the upper portion of the cigarette holder 60 may be designed to have a round-shaped cross section.

In the second embodiment, as shown in FIG.9, FIG. 10 and FIG. 13 to FIG. 17, the switch assembly further includes an elastic pad 74 disposed between the sealing block 76 and the air inlet 71. The elastic pad 74 may be made of edible silica gel and has a through hole 741 corresponding to the air inlet 71. A concave 72 is formed around the air inlet 71 for positioning the elastic pad 74, and a convex 742 corresponding to the concave 72 is provided at the bottom of the elastic pad 74. The through hole 741 extends through the convex 742.

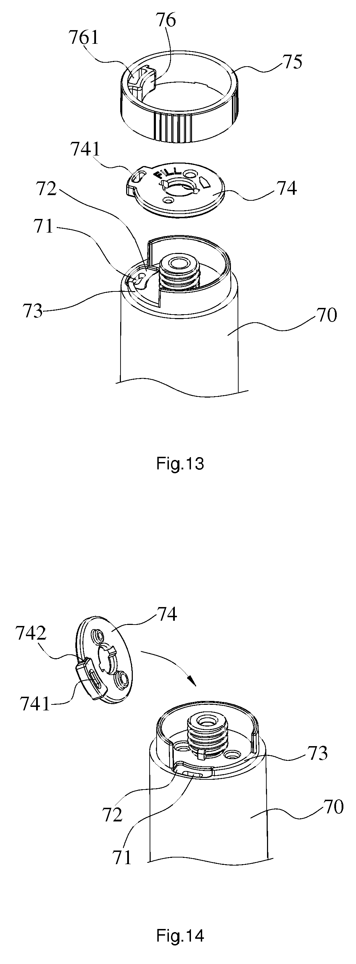

As shown in FIG. 13 to FIG. 17, the sealing block 76 and the round ring 75 form an integrated body and the sealing block 76 has a blind hole 761 formed on the upper surface thereof. The atomizer 70 has a limiting cylinder formed on the top thereof and the limiting cylinder has a notch 73 formed thereon for restricting the rotation range of the sealing block 76.

In the second embodiment, as shown in FIG.9, FIG. 10 and FIG. 18 to FIG. 20, the battery assembly includes a battery 90, a casing 91 for containing the battery 90 and a voltage adjusting unit which includes an adjustable resistor 81 in series with the battery 90 and an adjusting knob 83. By means of the adjusting knob 83, a resistance value of the adjustable resistor 81 is capable of being changed so as to adjust the voltage applied on the atomizer. The voltage adjusting unit is disposed below the battery 90.

As shown in FIG.9, FIG. 10 and FIG. 18 to FIG. 20, the voltage adjusting unit further includes a connecting sleeve 84, for determining a relative location between the adjustable resistor 81 and the adjusting knob 83, and a fixed round ring 85, by which the connecting sleeve 84 is fixed into the casing 91. The adjusting knob 83 has a central shaft 831 for connecting with the adjustable resistor 81, and the central shaft 831 is clamped by the connecting sleeve 84 and is capable of rotating with respect to the connecting sleeve 84. The adjustable resistor 81 is fixed on a circuit board 82 which is clamped by the connecting sleeve 84. The fixed round ring 85 is disposed on outside surface of the connecting sleeve 84 and fixed to inner surface of the casing 91 by an interference fit. The fixed round ring 85 is made of copper materials.

While the invention has been described in connection with what are presently considered to be the most practical and preferred embodiments, it is to be understood that the invention is not to be limited to the disclosed embodiments, but on the contrary, is intended to cover various modifications and equivalent arrangements.

## Claims

1. An electronic atomization device with an adjustable air inlet, comprising a cigarette holder (10; 60), an atomizer (20; 70) connected to the cigarette holder and a battery assembly (40) for supplying power to the atomizer, wherein further comprises an air inlet (21; 71) disposed at the upper portion of the atomizer for connecting the atomizer to outside world and a switch assembly (50) disposed between the atomizer and the cigarette holder for opening or closing the air inlet,
**characterized in that,** wherein the switch assembly comprises a round ring (51; 75) sandwiched between the atomizer and the cigarette holder and a sealing block (52; 76) fixed inside the round ring for opening or closing the air inlet, the round ring being coaxial with the atomizer, and when the round ring rotates with respect to the atomizer, the air inlet will be opened or closed by the sealing block which is driven by the round ring,
wherein the sealing block contacts with an upper surface of the air inlet and the upper surface of the air inlet is an inclined surface so that the sealing block is capable of applying pressing force onto the upper surface of the air inlet when the air inlet is closed completely by means of the sealing block.

2. The electronic atomization device according to claim 1, wherein the round ring has a flange (511) disposed therein and the sealing block is fixed on the flange.

3. The electronic atomization device according to claim 2, wherein the flange has a limiting notch (512) formed thereon and the atomizer has a limiting pillar (23) disposed at the top thereof and extending throughout the limiting notch.

4. The electronic atomization device according to claim 1, wherein the round ring has an inlet notch (514) formed at the bottom thereof and the air inlet connects to the outside world via the inlet notch.

5. The electronic atomization device according to claim 1, wherein the round ring has anti-slip strips (513) formed on an outside surface thereof.

6. The electronic atomization device according to claim 1, wherein the sealing block is an elastic sealing block.

7. The electronic atomization device according to claim 1, wherein the switch assembly further comprises an elastic pad (74) disposed between the sealing block and the air inlet, and the elastic pad has a through hole (741) corresponding to the air inlet.

8. The electronic atomization device according to claim 7, wherein a concave (72) is formed around the air inlet for positioning the elastic pad and a convex (742) corresponding to the concave is provided at the bottom of the elastic pad, and the through hole is extended through the convex.

9. The electronic atomization device according to claim 1, wherein the sealing block and the round ring form an integrated body and the sealing block has a blind hole (761) formed on an upper surface thereof.

10. The electronic atomization device according to claim 1, wherein the atomizer has a limiting cylinder formed on the top thereof and the limiting cylinder has a notch (73) formed thereon for restricting the rotation range of the sealing block.

11. The electronic atomization device according to claim 1, wherein the atomizer has an air passage (24) formed therein, by which the atomizer connects to the air inlet.

12. The electronic atomization device according to claim 11, wherein the air passage is a circular chamber.

13. The electronic atomization device according to claim 1, wherein the cigarette holder comprises a smoke channel (61), which is provided within the cigarette holder and connected to the atomizer, and two smoke outlets (62) connected to the smoke channel, and the smoke outlets are disposed at a sidewall of the cigarette holder.

## Patentansprüche

1. Elektronische Zerstäubungsvorrichtung mit einem einstellbaren Lufteinlass, umfassend einen Zigarettenhalter (10; 60), einen Zerstäuber (20; 70), der mit dem Zigarettenhalter verbunden ist, und eine Batterieanordnung (40) für die Energieversorgung des Zerstäubers, wobei sie ferner einen Lufteinlass (21; 71), der am oberen Teil des Zerstäubers zum Anschluss des Zerstäubers an die Außenwelt angeordnet ist, und eine Schalteranordnung (50), die zwischen dem Zerstäuber und dem Zigarettenhalter zum Öffnen oder Schließen des Lufteinlasses angeordnet ist, umfasst,
**dadurch gekennzeichnet, dass** die Schalteranordnung einen runden Ring (51; 75) im Sandwich zwischen dem Zerstäuber und dem Zigarettenhalter und einen Dichtungsblock (52; 76), welcher innerhalb des runden Rings zum Öffnen oder Schließen des Lufteinlasses befestigt ist, umfasst, wobei der runde Ring koaxial mit dem Zerstäuber ist, und wenn der runde Ring in Bezug auf den Zerstäuber rotiert, der Lufteinlass durch den Dichtungsblock, der durch den runden Ring angetrieben ist, geöffnet oder verschlossen wird,
wobei der Dichtungsblock in Kontakt mit einer oberen Fläche des Lufteinlasses ist und die obere Fläche des Lufteinlasses eine Schrägfläche ist, so dass der Dichtungsblock in der Lage ist, eine Anpresskraft auf die obere Fläche des Lufteinlasses auszuüben, wenn der Lufteinlass mittels des Dichtungsblocks vollständig verschlossen ist.

2. Elektronische Zerstäubungsvorrichtung nach Anspruch 1, wobei der runde Ring einen Flansch (511) aufweist, welcher darin angeordnet ist, und der Dichtungsblock am Flansch befestigt ist.

3. Elektronische Zerstäubungsvorrichtung nach Anspruch 2, wobei der Flansch eine darauf gebildete Begrenzungskerbe (512) aufweist und der Zerstäuber eine Begrenzungssäule (23) aufweist, welche an dessen Oberteil angeordnet ist und sich über die gesamte Begrenzungskerbe erstreckt.

4. Elektronische Zerstäubungsvorrichtung nach Anspruch 1, wobei der runde Ring eine an dessen Boden gebildete Einlass-Kerbe (514) aufweist und der Lufteinlass durch die Einlass-Kerbe mit der Außenwelt verbunden ist.

5. Elektronische Zerstäubungsvorrichtung nach Anspruch 1, wobei der runde Ring an einer Außenfläche ausgebildete Antirutsch-Streifen (513) aufweist.

6. Elektronische Zerstäubungsvorrichtung nach Anspruch 1, wobei der Dichtungsblock ein elastischer Dichtungsblock ist.

7. Elektronische Zerstäubungsvorrichtung nach Anspruch 1, wobei die Schalteranordnung ferner ein elastisches Pad (74) umfasst, das zwischen dem Dichtungsblock und dem Lufteinlass angeordnet ist, und das elastische Pad ein mit dem Lufteinlass übereinstimmendes Durchgangsloch (741) aufweist.

8. Elektronische Zerstäubungsvorrichtung nach Anspruch 7, wobei eine konkave Fläche (72) um den Lufteinlass zur Positionierung des elastischen Pads gebildet wird, und eine der konkaven Fläche entsprechenden konvexe Fläche (742) an der Unterseite des elastischen Pads vorhanden ist, und sich das Durchgangsloch durch die konvexe Fläche erstreckt.

9. Elektronische Zerstäubungsvorrichtung nach Anspruch 1, wobei der Dichtungsblock und der runde Ring einen integrierten Körper bilden und der Dichtungsblock ein Sackloch (761) aufweist, das an einer oberen Fläche davon gebildet ist.

10. Elektronische Zerstäubungsvorrichtung nach Anspruch 1, wobei der Zerstäuber einen an dessen Oberseite gebildeten Begrenzungszylinder aufweist und der Begrenzungszylinder eine darauf gebildete Kerbe (73) zum Beschränken des Rotationsbereichs des Dichtungsblocks aufweist.

11. Elektronische Zerstäubungsvorrichtung nach Anspruch 1, wobei der Zerstäuber einen darin gebildeten Luftdurchlass (24) aufweist, durch welchen der Zerstäuber mit dem Lufteinlass verbunden ist.

12. Elektronische Zerstäubungsvorrichtung nach Anspruch 11, wobei der Luftdurchlass eine kreisförmige Kammer ist.

13. Elektronische Zerstäubungsvorrichtung nach Anspruch 1, wobei der Zigarettenhalter einen Rauchkanal (61), welcher innerhalb des Zigarettenhalters vorhanden und mit dem Zerstäuber verbunden ist, und zwei Rauchausgänge (62), die mit dem Rauchkanal verbunden sind, umfasst, und die Rauchausgänge an einer Seitenwand des Zigarettenhalters angeordnet sind.

## Revendications

1. Dispositif d'atomisation électronique avec une entrée d'air déplaçable, comprenant un support de cigarette (10; 60), un pulvérisateur (20; 70) raccordé au support de cigarette et un montage de batterie (40) destiné à fournir de l'énergie au pulvérisateur, dans lequel il comprend en outre une entrée d'air (21 ; 71) disposée sur la partie supérieure du pulvérisateur pour assurer le raccordement du pulvérisateur avec le monde extérieur et un montage de commutateur (50) disposé entre le pulvérisateur et le support de cigarette pour l'ouverture ou la fermeture de l'entrée d'air,
**caractérisé en ce que** le montage de commutateur comprend un anneau rond (51 ; 75) en sandwich entre le pulvérisateur et le support de cigarette et un bloc d'étanchéité (52 ; 76) fixé à l'intérieur de l'anneau rond pour l'ouverture ou la fermeture de l'entrée d'air, l'anneau rond étant coaxial au pulvérisateur, et lorsque l'anneau rond tourne par rapport au pulvérisateur, l'entrée d'air est ouverte ou fermée par le bloc d'étanchéité entraîné par l'anneau rond,
dans lequel le bloc d'étanchéité est en contact avec une surface supérieure de l'entrée d'air et la surface supérieure de l'entrée d'air est une surface inclinée de manière à ce que le bloc d'étanchéité soit capable d'exercer une force de pression sur la surface supérieure de l'entrée d'air lorsque l'entrée d'air est complètement fermée au moyen du bloc d'étanchéité.

2. Dispositif d'atomisation électronique selon la revendication 1, dans lequel l'anneau rond présente une bride (511) disposée à l'intérieur et le bloc d'étanchéité est fixé sur la bride.

3. Dispositif d'atomisation électronique selon la revendication 2, dans lequel la bride présente une entaille limite (512) formée là-dessus et le pulvérisateur présente un pilier limite (23) disposé sur la partie supérieure de ce dernier et s'étendant à travers toute l'entaille limite.

4. Dispositif d'atomisation électronique selon la revendication 1, dans lequel l'anneau rond présente une entaille d'entrée (514) formée sur le fond de ce dernier et l'entrée d'air se raccorde au monde extérieur via l'entaille d'entrée.

5. Dispositif d'atomisation électronique selon la revendication 1, dans lequel l'anneau rond présente des bandes antiglissantes (513) formées sur une surface extérieure de ce dernier.

6. Dispositif d'atomisation électronique selon la revendication 1, dans lequel le bloc d'étanchéité est un bloc d'étanchéité élastique.

7. Dispositif d'atomisation électronique selon la revendication 1, dans lequel le montage commutateur comprend en outre un coussinet élastique (74) disposé entre le bloc d'étanchéité et l'entrée d'air, et le coussinet élastique présente un trou de passage (741) correspondant à l'entrée d'air.

8. Dispositif d'atomisation électronique selon la revendication 7, dans lequel une surface concave (72) est formée autour de l'entrée d'air pour assurer le positionnement du coussinet élastique et une surface convexe (742) correspondant à la surface concave est prévue sur la partie inférieure du coussinet élastique, et le trou de passage s'étend à travers la surface convexe.

9. Dispositif d'atomisation électronique selon la revendication 1, dans lequel le bloc d'étanchéité et l'anneau rond forment un corps intégré et le bloc d'étanchéité présente un trou borgne (761) formé sur une surface supérieure de ce dernier.

10. Dispositif d'atomisation électronique selon la revendication 1, dans lequel le pulvérisateur présente un cylindre limite formé sur le haut de ce dernier et le cylindre limite présente une entaille (73) formée sur ce dernier pour restreindre la plage de rotation du bloc d'étanchéité.

11. Dispositif d'atomisation électronique selon la revendication 1, dans lequel le pulvérisateur présente un passage d'air (24) formé dans ce dernier via lequel le pulvérisateur se raccorde à l'entrée d'air.

12. Dispositif d'atomisation électronique selon la revendication 11, dans lequel le passage d'air est une chambre circulaire.

13. Dispositif d'atomisation électronique selon la revendication 1, dans lequel le support de cigarette comprend un canal de fumée (61) qui comporte le support de cigarette et est raccordé au pulvérisateur, et deux sorties de fumée (62) raccordées au canal de fumée, et les sorties de fumée sont disposées sur une paroi latérale du support de cigarette.
